# EUROPEAN PATENT SPECIFICATION

(11) **EP 3 311 732 B1**
(45) Date of publication and mention of the grant of the patent: **09.03.2022**
(21) Application number: 15862124.3
(22) Date of filing: 21.10.2015
(51) Int. Cl.: A61B 1/227

(54) **VISUAL INSPECTION ASSISTING EQUIPMENT**
ENDOSKOPHILFSVORRICHTUNG
ÉQUIPEMENT D'AIDE À L'INSPECTION VISUELLE

(30) Priority: 19.06.2015 CN 201510345139
(43) Date of publication of application: 25.04.2018
(73) Proprietor: BOE Technology Group Co., Ltd., Beijing 100015 (CN); BOE Optical Science And Technology Co., Ltd., Suzhou, Jiangsu 215021 (CN)
(72) Inventor: CAO, Chunlei, Beijing 100176 (CN)
(74) Representative: Klunker IP Patentanwälte PartG mbB
(86) International application number: PCT/CN2015/092412
(87) International publication number: WO 2016/201841

(56) References cited:
- CN-A- 101 451 683
- CN-A- 102 475 531
- CN-A- 104 921 692
- CN-U- 201 958 856
- CN-U- 204 318 706
- CN-U- 204 698 496
- US-A1- 2004 155 565
- US-A1- 2007 255 108
- US-A1- 2009 253 962
- US-A1- 2011 009 694
- US-A1- 2013 281 790
- US-B1- 6 621 973
- US-B2- 7 371 066

## Description

### TECHNICAL FIELD

The present invention relates to an endoscope auxiliary device.

### BACKGROUND

Ear clearing is a regular behavior in our daily life. A conventional ear picker is mainly made of metals, plastics, woods or bamboos. Ear canal is tortuous and deep, light cannot irradiate to the ear canal, so that it is very inconvenient to clear earwax.

In a conventional ear picker, the inconvenience described above is resolved to a certain degree by providing a light source on the ear picker, however, there is insufficient utilization ratio of illumination of the light source. So it is easy to form defects, such as light spot, light shadow and others on the object, and the observing result is affected thereby; while, if a light source of high brightness is used, a light spot may be formed, and the high brightness may be harm to the eyes of a user.

US 2011/0009694 A1 describes a hand-held minimally dimensioned diagnostic device having an integrated distal end visualization element. In addition to the distal end integrated visualization sensor, devices of the US 2011/0009694 A1 also include a distal end integrated non-visualization sensor. The one or more non-visualization sensors are sensors that are configured to obtain non-visual data from a target location.

US 2013/281790 A1 describes medical retractors including light guides made of cyclo-olefin polymer and copolymer.

US 2007/255108 A1 describes a water-resistant LED pocket otoscope.

US 6,621,973 A1 describes a light guide with protective outer sleeve.

### SUMMARY

It is an object of the invention to provide an endoscope auxiliary device that may solve one or more problem present in the art.

The object is achieved by the features of the independent claim.

Further embodiments are defined in the corresponding dependent claims.

### BRIEF DESCRIPTION OF THE DRAWINGS

Embodiments of the present disclosure will be described in detail hereinafter in conjunction with accompanying drawings to allow one of ordinary skill in the art to understand the present disclosure more clearly, in which:
FIG. 1 is a structural schematic diagram of an endoscope auxiliary device of an embodiment of the present disclosure;
FIG. 2 is a structural schematic diagram of a control system of the endoscope auxiliary device of an embodiment of the present disclosure.

### DETAILED DESCRIPTION

The technical solutions of the embodiments will be described in a clearly and fully understandable way in connection with the drawings related to the embodiments of the disclosure. Apparently, the described embodiments are just a part but not all of the embodiments of the disclosure. Based on the described embodiments herein, those skilled in the art can obtain other embodiment(s), without any inventive work, which should be within the scope of the disclosure.

Unless otherwise defined, all the technical and scientific terms used herein have the same meanings as commonly understood by an ordinary skill in the art to which the present invention belongs. The terms, such as "first," "second" or the like, which are used in the description and the claims of the present application, are not intended to indicate any sequence, amount or importance, but for distinguishing various components. Also, the terms, such as "a/an," "the" or the like, are not intended to limit the amount, but for indicating the existence of at lease one. The terms, such as "comprise/comprising," "include/including" or the like, are intended to specify that the elements or the objects stated before these terms encompass the elements or the objects and equivalents thereof listed after these terms, but not preclude other elements or objects. The terms, such as "On," "under," or the like, are only used to indicate relative position relationship, and when the position of the object which is described is changed, the relative position relationship may be changed accordingly.

To reduce light spot and light shadow formed on an object, and improve an accuracy of a result of an endoscope observation, an embodiment of the present disclosure provides an endoscope auxiliary device. To make the objective, the technical solution and the advantages of the present disclosure clearer, the present disclosure shall be further described in detail hereinafter in conjunction with the examples.

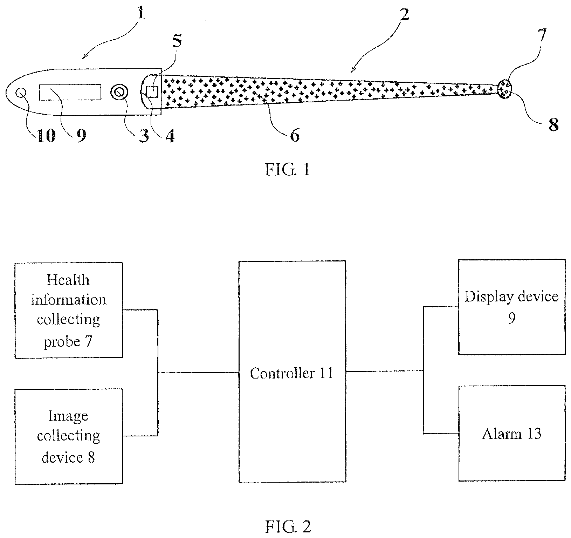

As shown in FIG. 1, an endoscope auxiliary device provided by the embodiment of the present disclosure includes an operating handle 1 and a probing body 2 connected with each other. A switching device 3 is arranged on the operating handle 1. Optical mesh points 6 are provided on a surface of the probing body 2. A light source 5 controlled by the switching device 3 and a reflective housing 4 for reflecting light of the light source 5 into the probing body 2 are arranged on an end of the probing body 2 close to the operating handle 1.

A type of the endoscope auxiliary device may be, but not limited to, for example, such as an endoscope auxiliary device for nasal cavity, an endoscope auxiliary device for ear canal, a laryngendoscope auxiliary device, or an endoscope auxiliary device for mouth. The endoscope auxiliary device shown in FIG. 1, for example, is an endoscope auxiliary device for ear canal, which has a function of ear picker. An ear spoon 12 is arranged on an end of the probing body 2 away from the operating handle 1.

A light-emitting diode has advantages, such as energy-saving, high brightness, low heat, soft light and the like, for example, the light source 5 adopts a light source of the light-emitting diode. Structures of the reflective housing 4, the probing body 2 and the light source 5 are not limited. As shown in FIG. 1, in the embodiment, the light source 5 is embedded in an end of the probing body 2 close to the operating handle 1, and the reflective housing 4 is arranged on a surface of the end of the probing body 2 close to the operating handle 1. The reflective housing 4 and the end of the probing body 2 close to the operating handle 1 are fixed in the operating handle 1. The reflective housing 4 usually includes a base layer and a reflective layer. The reflective layer is arranged to face the light source 5, and the reflective layer may effectively reflect light of the light source 5 into the probing body 2.

For example, the probing body 2 is made of material of polymethyl methacrylate; luminance of the light source may be improved by using the material of polymethyl methacrylate, and light scattering may be suppressed, so that a utilization ratio of the light source may be improved. The light spot and light shadow formed in the ear canal may be reduced, and the probing body made of the material of polymethyl methacrylate has good toughness, which may not be easily broken, so as to reduce risk of breaking at the time of cleaning ear.

In an embodiment of the present disclosure, the reflective housing 4 may reflect light emitted by the light source 5 into the probing body 2, and the reflected light is scattered in various directions by using an optical mesh point 6 on the surface of the probing body 2 and conditions of total reflection is destroyed, so that the emitted light is more uniform, and a utilization ratio of the light source may be improved. The sufficient light is irradiated onto the object, and the light spot and light shadow formed on the object are reduced, as well as an accuracy of a result of an endoscope observation is improved. By using the endoscope auxiliary device shown in FIG. 1, the ear canal is probed and the earwax is cleaned, and the observation and operation are more convenient and accurate.

In other examples of the present disclosure, which do not fall under the scope of the claimed subject-matter, an endoscope auxiliary device for oral cavity, a magnifying glass may be arranged on an end of the probing body away from the operating handle. When a mouth is observed by using the mouth endoscope, the magnifying glass may magnify a part to be observed in the mouth, so that a user may observe the part to be observed in the mouth more clearly.

For example, refer to FIG. 1 and FIG. 2 again, a health information collecting probe 7 is provided on an end of the probing body 2 away from the operating handle 1, and a controller 11 signally communicated with the health information collecting probe 7 is arranged on the operating handle 1, and the controller 11 performs data processing on the health information, and sends the health information to the display device 9 for displaying. When probing the ear canal or cleaning the earwax, the health information collecting probe 7 accurately monitors the health information of the ear canal in real time, and sends the health information to the controller 11. The controller 11 processes and sends the health information to the display device 9 for displaying, so that the user may conveniently and quickly know a health condition of the ear canal by viewing the display device 9.

In the embodiment of the present disclosure, once the health information collecting probe 7 detects abnormality in the health information, for example, exceeding a predetermined value, continuously increasing, continuously decreasing or the like, the controller 11 may trigger the display device 9 to display an alarm. For example, the display device 9 may display messages such as "hyperthermia or higher body temperature", "higher humidity in the ear canal" or the like, so that the user may be prompted that the ear canal has probably had a pathological change.

The health information collecting probe 7 is a humidity collecting probe or a humiture collecting probe. The health information collecting probe 7 may be, but not limited to, a collecting probe of a resistive type, a capacitive type or an infrared type, or the like. In the present invention, humiture or humidity of the ear canal and the earwax are monitored in real time by using the health information collecting probe 7. Since the temperature and humidity reflect a result of metabolism of an organism, and a normal temperature and a humidity are necessary conditions for the organism to perfonn normal functions, a health condition of the ear canal may be better reflected by using the humiture collecting probe.

As further shown in FIG. 1 and FIG. 2, an image collecting device 8 is provided on an end of the probing body 2 away from the operating handle 1, and the controller 11 is signally communicated with the image collecting device 8. The controller 11 performs data processing on the image collected by the image collecting device 8, and sends the image to the display device 9 for displaying. The user may conveniently observe images of an object by viewing the display device 9, which is benefit to accurately determining a probable focus of the object in time.

According to the present disclosure, at least one of a bluetooth transmission device, a USB interface and a video transmission device may be further arranged on the operating handle. With those transmission interfaces, data information may be transferred to an external device for further analyzing. For example, in the embodiment shown in FIG. 1, a video transmission interface 10 is arranged on the operating handle 1 of the endoscope auxiliary device.

As shown in FIG. 2, in other embodiments of the present disclosure, an alarm 13 may be further arranged on the operating handle. When an abnormality is presented in the health information collected by the health information collecting probe 7, for example, exceeding a predetermined value, continuously increasing, continuously decreasing or the like, and the control device may trigger the alarm 13 to give an alarm, to prompt the user that the object may have a pathological change.

## Claims

1. An endoscope auxiliary device, comprising an operating handle (1) and a probing body (2) connected with each other, wherein
a switching device (3) is provided on the operating handle (1);
optical mesh points (6) are provided on a surface of the probing body (2);
a light source (5) is controlled by the switching device (3) and a reflective housing (4) is configured to reflect light of the light source (5) into the probing body (2) provided on one end of the probing body (2) close to the operating handle (1); and
a health information collecting probe (7) including a humidity collecting probe or a humiture collecting probe provided on another end of the probing body (2) away from the operating handle (1) and configured to collect and monitor health
information on humidity or humiture of an ear canal and earwax in real time;
a controller (11) in communication with the humidity collecting probe or the humiture collecting probe and configured to process the health information on the humidity or the humiture of the ear canal and the earwax received by the health information collecting probe (7); and
a display device (9) in communication with the controller (11) wherein the controller (11) is configured to send the processed health information to the display device (9), wherein the display device (9) is configured to display the processed health information on humidity or humiture of the ear canal and the earwax received from the controller (11).

2. The endoscope auxiliary device according to claim 1, wherein the light source (5) is embedded in an end of the probing body (2) close to the operating handle (1), the reflective housing (4) is provided on the surface of the end of the probing body (2) close to the operating handle (2), and the reflective housing (4) and the end of the probing body (2) close to the operating handle (1) are fixed in the operating handle (1).

3. The endoscope auxiliary device according to claim 1 or claim 2, wherein a magnifying device is provided on the another end of the probing body (2) away from the operating handle (1).

4. The endoscope auxiliary device according to any one of claims 1 to 3, wherein the light source (5) includes a light-emitting diode, and the probing body (2) includes a probing body made of material of polymethyl methacrylate.

5. The endoscope auxiliary device according to claim 1, wherein the controller (11) is further configured to trigger the display device (9) to display an alarm when the health information on the humidity or the humiture does not meet predetermined conditions.

6. The endoscope auxiliary device according to claim 1, wherein an alarm (13) is further provided on the operating handle (1), the controller (11) being configured to trigger the alarm (13) to give an alarm when the health information on the humidity or the humiture does not meet predetermined conditions

7. The endoscope auxiliary device according to any one of claims 1 to 6, wherein an image collecting device (8) is further provided on the end of the probing body (2) away from the operating handle (1); and
the controller (11) is further signally communicated with the image collecting device (8), the controller (11) being configured to perform data processing on the image collected by the image collecting device (8), and sending the image to the display device (9) for displaying.

8. The endoscope auxiliary device according to any one of claims 1 to 7, wherein a Bluetooth transmission device is further provided on the operating handle (1).

## Patentansprüche

1. Endoskop-Hilfsvorrichtung mit einem Bediengriff (1) und einem Sondierungskörper (2), die miteinander verbunden sind, wobei
eine Schaltvorrichtung (3) an dem Bediengriff (1) vorgesehen ist;
auf einer Oberfläche des Sondierungskörpers (2) optische Gitterpunkte (6) vorgesehen sind;
eine Lichtquelle (5) durch die Schaltvorrichtung (3) gesteuert wird und ein reflektierendes Gehäuse (4) so konfiguriert ist, dass es Licht der Lichtquelle (5) in den Sondierungskörper (2) reflektiert, der an einem Ende des Sondierungskörpers (2) nahe dem Bediengriff (1) vorgesehen ist; und
eine Sonde (7) zum Sammeln von Gesundheitsinformationen, die eine Sonde zum Sammeln von Feuchtigkeit oder eine Sonde zum Sammeln von Humiture enthält, die an einem anderen Ende des Sondierungskörpers (2) entfernt von dem Bediengriff (1) vorgesehen ist und so konfiguriert ist, dass sie Gesundheitsinformationen über Feuchtigkeit oder Humiture eines Gehörgangs und Ohrenschmalz in Echtzeit sammelt und überwacht;
eine Steuereinheit (11), die mit der Sonde zum Sammeln von Feuchtigkeit oder der Sonde zum Sammeln von Humiture in Verbindung steht und so konfiguriert ist, dass sie die von der Sonde (7) zum Sammeln von Gesundheitsinformationen empfangenen Gesundheitsinformationen über die Feuchtigkeit oder die Humiture des Gehörgangs und des Ohrenschmalzes verarbeitet; und
eine Anzeigevorrichtung (9), die mit der Steuereinheit (11) in Verbindung steht, wobei die Steuereinheit (11) so konfiguriert ist, dass sie die verarbeiteten Gesundheitsinformationen an die Anzeigevorrichtung (9) sendet, wobei die Anzeigevorrichtung (9) so konfiguriert ist, dass sie die von der Steuereinheit (11) empfangenen verarbeiteten Gesundheitsinformationen über die Feuchtigkeit oder den Humiture des Gehörgangs und das Ohrenschmalz anzeigt.

2. Endoskop-Hilfsvorrichtung nach Anspruch 1, wobei die Lichtquelle (5) in ein Ende des Sondierungskörpers (2) nahe dem Bediengriff (1) eingebettet ist, das reflektierende Gehäuse (4) auf der Oberfläche des Endes des Sondierungskörpers (2) nahe dem Bediengriff (2) vorgesehen ist und das reflektierende Gehäuse (4) und das Ende des Sondierungskörpers (2) nahe dem Bediengriff (1) in dem Bediengriff (1) befestigt sind.

3. Endoskop-Hilfsvorrichtung nach Anspruch 1 oder Anspruch 2, wobei an dem anderen, vom Bediengriff (1) abgewandten Ende des Sondierungskörpers (2) eine Vergrößerungsvorrichtung vorgesehen ist.

4. Endoskop-Hilfsgerät nach einem der Ansprüche 1 bis 3, wobei die Lichtquelle (5) eine Leuchtdiode umfasst und der Sondierungskörpers (2) einen Sondierungskörper aus Polymethylmethacrylat-Material umfasst.

5. Endoskop-Hilfsvorrichtung nach Anspruch 1, wobei die Steuereinheit (11) ferner so konfiguriert ist, dass sie die Anzeigevorrichtung (9) zur Anzeige eines Alarms veranlasst, wenn die Gesundheitsinformationen über die Feuchtigkeit oder die Humiture nicht den vorgegebenen Bedingungen entsprechen.

6. Endoskop-Hilfsvorrichtung nach Anspruch 1, wobei ferner ein Alarm (13) an dem Bediengriff (1) vorgesehen ist, wobei die Steuerung (11) so konfiguriert ist, dass sie den Alarm (13) auslöst, um einen Alarm auszugeben, wenn die Gesundheitsinformationen über die Feuchtigkeit oder die Humiture nicht den vorbestimmten Bedingungen entsprechen.

7. Endoskop-Hilfsvorrichtung nach einem der Ansprüche 1 bis 6, wobei eine Bildsammelvorrichtung (8) an dem von dem Bediengriff (1) entfernten Ende des Sondierungskörpers (2) vorgesehen ist; und
die Steuerung (11) ferner mit der Bildsammelvorrichtung (8) signalmäßig kommuniziert, wobei die Steuerung (11) so konfiguriert ist, dass sie eine Datenverarbeitung des von der Bildsammelvorrichtung (8) gesammelten Bildes durchführt und das Bild zur Anzeige an die Anzeigevorrichtung (9) sendet.

8. Endoskop-Hilfsgerät nach einem der Ansprüche 1 bis 7, wobei an dem Bediengriff (1) ferner eine Bluetooth-Übertragungseinrichtung vorgesehen ist.

## Revendications

1. Dispositif auxiliaire d'endoscope, comprenant une poignée de commande (1) et un corps de sondage (2) qui sont connectés l'un à l'autre, dans lequel :
un dispositif de commutation (3) est prévu sur la poignée de commande (1) ;
des points de maillage optique (6) sont prévus sur une surface du corps de sondage (2) ;
une source de lumière (5) est commandée par le dispositif de commutation (3) et un logement réfléchissant (4) est configuré pour réfléchir la lumière de la source de lumière (5) à l'intérieur du corps de sondage (2) et est prévu sur une extrémité du corps de sondage (2) à proximité de la poignée de commande (1) ; et
une sonde de collecte d'information de santé (7) qui inclut une sonde de collecte d'humidité ou une sonde de collecte de combinaison humidité-température est prévue sur une autre extrémité du corps de sondage (2) à distance de la poignée de commande (1) et est configurée pour collecter et surveiller une information de santé relative à l'humidité ou à une combinaison humidité-température d'un conduit auditif et du cérumen en temps réel ;
un contrôleur (11) est en communication avec la sonde de collecte d'humidité ou la sonde de collecte de combinaison humidité-température et est configuré pour traiter l'information de santé relative à l'humidité ou à la combinaison humidité-température du conduit auditif et du cérumen qui est reçue par la sonde de collecte d'information de santé (7) ; et
un dispositif d'affichage (9) est en communication avec le contrôleur (11), dans lequel le contrôleur (11) est configuré pour envoyer l'information de santé traitée sur le dispositif d'affichage (9), dans lequel le dispositif d'affichage (9) est configuré pour afficher l'information de santé traitée relative à l'humidité ou à la combinaison humidité-température du conduit auditif et du cérumen qui est reçue depuis le contrôleur (11).

2. Dispositif auxiliaire d'endoscope selon la revendication 1, dans lequel la source de lumière (5) est intégrée dans une extrémité du corps de sondage (2) à proximité de la poignée de commande (1), le logement réfléchissant (4) est prévu sur la surface de l'extrémité du corps de sondage (2) à proximité de la poignée de commande (1), et le logement réfléchissant (4) et l'extrémité du corps de sondage (2) à proximité de la poignée de commande (1) sont fixés dans la poignée de commande (1).

3. Dispositif auxiliaire d'endoscope selon la revendication 1 ou la revendication 2, dans lequel un dispositif d'agrandissement est prévu sur l'autre extrémité du corps de sondage (2) à distance de la poignée de commande (1).

4. Dispositif auxiliaire d'endoscope selon l'une quelconque des revendications 1 à 3, dans lequel la source de lumière (5) inclut une diode électroluminescente, et le corps de sondage (2) inclut un corps de sondage réalisé en un matériau de méthacrylate de polyméthyle.

5. Dispositif auxiliaire d'endoscope selon la revendication 1, dans lequel le contrôleur (11) est en outre configuré pour déclencher le dispositif d'affichage (9) pour afficher une alarme lorsque l'information de santé relative à l'humidité ou à la combinaison humidité-température ne satisfait pas des conditions prédéterminées.

6. Dispositif auxiliaire d'endoscope selon la revendication 1, dans lequel une alarme (13) est en outre prévue sur la poignée de commande (1), le contrôleur (11) étant configuré pour déclencher l'alarme (13) pour produire une alarme lorsque l'information de santé relative à l'humidité ou à la combinaison humidité-température ne satisfait pas des conditions prédéterminées.

7. Dispositif auxiliaire d'endoscope selon l'une quelconque des revendications 1 à 6, dans lequel un dispositif de collecte d'image (8) est en outre prévu sur l'extrémité du corps de sondage (2) à distance de la poignée de commande (1) ; et
le contrôleur (11) est en outre en communication en termes de signaux avec le dispositif de collecte d'image (8), le contrôleur (11) étant configuré pour réaliser un traitement de données sur l'image qui est collectée par le dispositif de collecte d'image (8) et envoyant l'image sur le dispositif d'affichage (9) pour son affichage.

8. Dispositif auxiliaire d'endoscope selon l'une quelconque des revendications 1 à 7, dans lequel un dispositif de transmission Bluetooth est en outre prévu sur la poignée de commande (1).
